# EUROPÄISCHE PATENTSCHRIFT

(11) **EP 1 429 789 B1**
(45) Veröffentlichungstag und Bekanntmachung des Hinweises auf die Patenterteilung: **14.05.2008**
(21) Anmeldenummer: 02800120.4
(22) Anmeldetag: 25.09.2002
(51) Int. Cl.: A61K 31/702, A61K 31/715, A61P 31/00

(54) **ANTIINFEKTIVE KOHLENHYDRATE**
ANTI-INFECTIOUS CARBOHYDRATES
GLUCIDES ANTI-INFECTIEUX

(30) Priorität: 25.09.2001 DE 10147100
(43) Veröffentlichungstag der Anmeldung: 23.06.2004
(73) Patentinhaber: N.V. Nutricia, 2712 HM Zoetermeer (NL)
(72) Erfinder: STAHL, Bernd, 61191 Rosbach (DE); FINKE, Berndt, 32049 Herford (DE); SCHMITT, Joachim, 63768 Hösbach (DE); GOEBEL, Werner, 97218 Gerbrunn (DE); SLAGHUIS, Jörg, 97082 Würzburg (DE)
(74) Vertreter: Köster, Hajo
(86) Internationale Anmeldenummer: PCT/EP2002/010761
(87) Internationale Veröffentlichungsnummer: WO 2003/028738

(56) Entgegenhaltungen:
- WO-A-94/18986
- DE-A- 10 104 055
- DE-A- 19 701 382
- US-A- 6 146 670
- PATENT ABSTRACTS OF JAPAN vol. 1996, no. 06, 28. Juni 1996 (1996-06-28) & JP 08 038064 A (MEIJI SEIKA KAISHA LTD), 13. Februar 1996 (1996-02-13)
- KUNZ, C.: "Komplexe Oligosaccharide in der Säuglingsernährung" MONATSSCHR KINDERHEILKD, Bd. 149, 1998, Seiten 49-56, XP002235104
- CHATURVEDI P ET AL: "MILK OLIGOSACCHARIDE PROFILES BY REVERSED-PHASE HPLC OF THEIR PERBENZOYLATED DERIVATIVES" ANALYTICAL BIOCHEMISTRY, ACADEMIC PRESS, SAN DIEGO, CA, US, Bd. 251, Nr. 1, 15. August 1997 (1997-08-15), Seiten 89-97, XP000917925 ISSN: 0003-2697

## Beschreibung

Die Erfindung betrifft die Verwendung von neutralen geradkettigen oder verzweigten Oligosacchariden zur Verhinderung der Invasion und Infektion von Säugerzellen durch Pathogene und zur Bekämpfung von durch derartige Pathogene verursachten Erkrankungen und diese Oligosaccharide enthaltende Nahrungs-, diätetische und pharmazeutische Mittel.

Die Adhäsion sowohl pathogener Organismen als auch zellschädigender Substanzen an die Oberfläche von Säugerzellen ist der erste Schritt und eine unabdingbare Voraussetzung für eine Infektion bzw. Schädigung der Zelle. Die Interaktion zwischen den Pathogenen und den Zellen kommt durch eine Ligand-Rezeptor-Beziehung zustande. Glycostrukturen spielen bei diesen Beziehungen bzw. Wechselwirkungen eine wichtige Rolle.

Eine Möglichkeit, derartige Ligand-Rezeptor-Beziehungen zu beeinflussen, besteht in der Blockierung und/oder der strukturellen Veränderung der jeweiligen Rezeptoren auf der Zelloberfläche oder der Liganden.

Verschiedene Kohlenhydratmischungen haben sich in spezifischen Testsystemen als sehr wirksam erwiesen, die Adhäsion beispielsweise von Mikroorganismen an die Zelloberfläche zu vermindern oder ganz zu verhindern, man vergleiche: Kunz, C.; Rudloff, S. Acta Paediatr. 1993, 82, 903-912. Andere Substanzen wie etwa die Lewis-Strukturen als Kohlenhydrat-Liganden der Selektine (Adhäsionsproteine auf Endothelien und Lymphocyten), modulieren die Interaktion der Lymphocyten mit dem Endothel beispielsweise im Rahmen von Rolling, Homing und der

Invasion bei entzündlichen Prozessen (Norman, K.E.; Anderson, G.P.; Kolb, H.C.; Ley, K.; Ernst, B. Blood 1998, 91, 475-483). Eine weitere wichtige physiologische Rolle im Zusammenhang sowohl mit grundlegenden zellulären Funktionen als auch mit spezifischen Funktionen wie Zelladhäsion, Migration, Chemotaxis, Proliferation, Apoptose, Neuritenwachstum erfüllen die Galactose-erkennenden Lektine, die Galectine. (Cooper DN & Barondes SH, Glycobiology 1999 9 (10) 979-984). Für den Nematoden C. elegans konnte gezeigt werden, dass dessen Galectin LEC-1 verschiedene Galactose enthaltende Oligosaccharid-Derivate mit unterschiedlicher Spezifität binden kann. (Arata Y. Hirabayashi J. Kasai K, JBC, 2001:276, 5, 3068-3077). In einem Mäusemodell konnte durch den Einsatz von Galactose spezifischen Lectinen die Letalität einer experimentellen Listeriose verringert werden (Stoffel B, Beuth J, Pulverer G, Zentralbl. Bakteriol. 1996, 284:439-442). Die Adhärenz von Mikroorganismen an Wirtszellen kann aber auch der Auslöser von Signalkaskaden sowohl in den körperfremden pathogenen als auch in den körpereigenen Zellen sein.

Eine andere Möglichkeit besteht darin, auf molekularbiologischer Ebene eine Beeinflussung zellulärer Prozesse zu erreichen. Dies kann dazu führen, dass etwa bei den Säugerzellen Abwehrmechanismen ausgelöst werden oder bei den pathogenen Mikroorganismen die Expression von Virulenzmechanismen (z.B. Abschalten von Virulenzgenen bei Bakterien durch Blockierung zentraler Regulatoren) verringert oder verhindert werden. Auf diese Weise kann die Expression bestimmter Oberflächenstrukturen von pathogenen Listerien, die für die Invasion in Wirtszellen verantwortlich sein, durch bestimmte Kohlenhydrate wie Cellobiose erfolgreich gehemmt werden (Park SF Kroll RH Mol Microbiol 1993 8:653:661; WO-A 94/02586).

Aufgabe der vorliegenden Erfindung ist es, einen Weg aufzuzeigen, wie mit Hilfe von Oligosacchariden die Invasion und Infektion von Säugerzellen durch Listerien verringert oder verhindert werden kann und durch derartige Listerien verursachte Erkrankungen wirksam bekämpft werden können.

Gelöst wird diese Aufgabe durch die Lehre der Ansprüche.

Erfindungsgemäße werden spezielle Oligosaccharide zur Lösung der erfindungsgemäßen Aufgabe zur Anwendung gebracht. Diese Oligosaccharide werden nachstehend als erfindungsgemäße Oligosaccharide bezeichnet.

Erfindungsgemäß können sowohl einzelne erfindungsgemäße Oligosaccharide alleine oder mehrere erfindungsgemäße Oligosaccharide in Mischung miteinander eingesetzt werden. Ferner ist es möglich, ein erfindungsgemäßes Oligosaccharid oder mehrere erfindungsgemäße Oligosaccharide oder auch eine Mischung von zahlreichen erfindungsgemäßen Oligosacchariden zusammen mit anderen, nicht zu den erfindungsgemäßen Oligosacchariden zählenden Kohlenhydraten in Form einer Kohlenhydratmischung einzusetzen.

Die erfindungsgemäßen Oligosaccharide weisen eine Grundeinheit der folgenden allgemeinen Formel lauf: 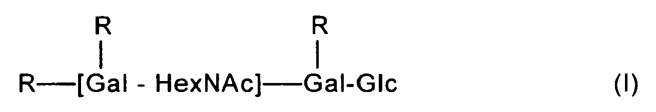 dabei bedeutet Gal eine Galactose-Monosaccharid-Einheit, Glc eine Glucose-Monosaccharid-Einheit und HexNAc eine N-acetylierte Galactosamin- oder Glucosamin-Monosaccharid-Einheit (kann auch abgekürzt werden als GalNAc bzw. GlcNAc).

Die Reste R in der allgemeinen Formel I müssen nicht vorhanden sein, wobei ein Wasserstoffatom im Rahmen der vorliegenden Unterlagen nicht als ein Rest angesehen wird. Einfach aufgebaute erfindungsgemäße Oligosaccharide sind somit solche der folgenden allgemeinen Formel

[Gal - HexNAc]―Gal-Glc.

Zu diesen Oligosacchariden zählt die erfindungsgemäß bevorzugte Lacto-N-tetraose (Gal β1-3GlcNAcβ 1-3Galβ 1-4Glc, LNT).

Bei letzteren Oligosacchariden stellt die [Gal - HexNAc] - Einheit eine endständige Einheit dar. Diese kann an der Galactose-Monosaccharid-Einheit (Gal) mit einer Deoxyhexose (vorzugsweise α1-2 glycosidisch) oder mit zwei Deoxyhexosen verknüpft sein. Der Rest R oder die Reste R in der allgemeinen Formel I kann/können in diesem Fall somit einen derartigen Deoxyhexose-Rest bedeuten, bei dem oder denen es sich bevorzugt um einen Rhamnose- und Fucose-Rest handelt.

Zu letzteren Verbindungen zählt die erfindungsgemäß bevorzugte Lacto-N-tetraose, die in α1-2 glycosidischer Verknüpfung an der endständigen Galactose-Monosaccharid-Einheit der [Gal - HexNAc] - Einheit durch Fucose derivatisiert ist; es handelt sich dabei um Lacto-N-fucopentaose I (LNFP I).

Die Reste R der Grundeinheit der allgemeinen Formel I können auch Verknüpfungsstellen zu weiteren [Gal - HexNAc] - Einheiten der folgenden allgemeinen Formel II darstellen:

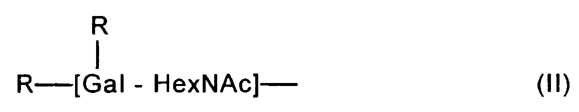

Die Reste R dieser direkt an die Grundeinheit der allgemeinen Formel I gebundenen [Gal - HexNAc] - Einheiten der allgemeinen Formel II (Es können ein oder zwei Reste R pro Einheit vorhanden sein) können wiederum Verknüpfungsstellen zu weiteren [Gal - HexNAc] - Einheiten der allgemeinen Formel II bedeuten, welche weiteren Einheiten dann indirekt an die Grundeinheit der allgemeinen Formel I gebunden sind. Auch die weiteren [Gal - HexNAc] - Einheiten der allgemeinen Formel II können dann wieder derartige Verknüpfungsstellen aufweisen usw. Die [Gal - HexNAc] - Einheiten sind dabei über eine β 1-3 oder β 1-6 glykosidische Verknüpfung verbunden. Zu diesen Oligosacchariden zählen beispielsweise diejenigen der folgenden allgemeinen Formeln, um nur einige zu nennen: 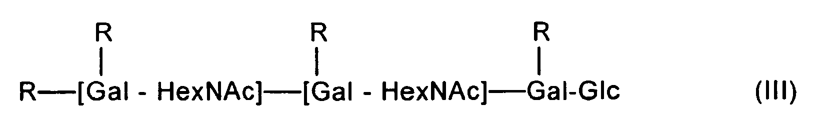 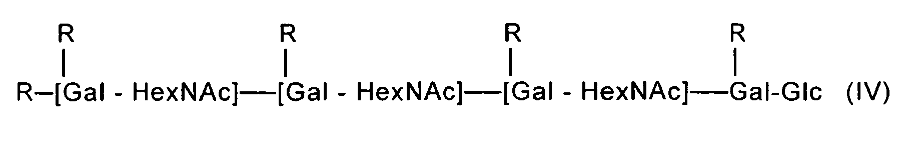 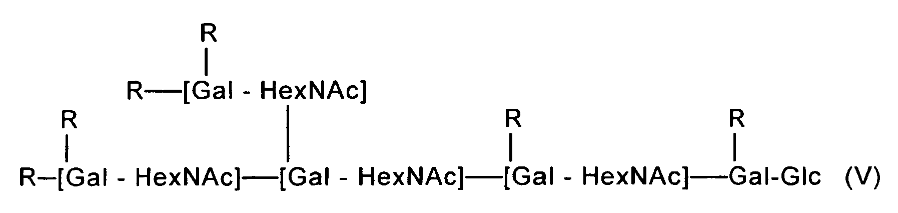 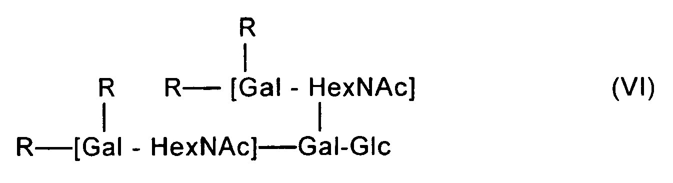 und

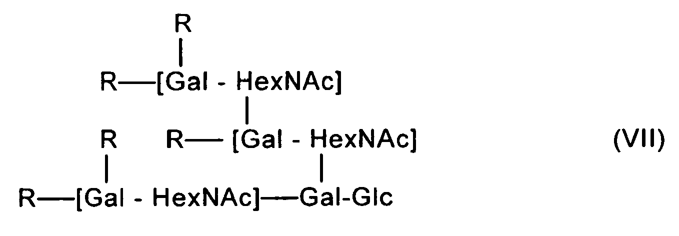

Bei diesen Formeln besitzen die Reste R die oben angegebenen Bedeutungen. So können beispielsweise alle Reste R nicht vorhanden sein. Ferner kann einer oder können beide der Reste R oder können auch alle Reste R eine Verknüpfung zur nächsten [Gal - HexNAc] - Einheit darstellen, die wiederum jeweils einen Rest R oder zwei Reste R aufweisen können oder auch keinen derartigen Rest R besitzen. Insgesamt können bis zu 20 [Gal - HexNAc] - Einheiten und somit 1, 2, 3, 4, 5, 6, 7, 8, 9, 10, 11, 12, 13, 14, 15, 16, 17, 18, 19 und 20 derartige Einheiten in den erfindungsgemäßen Oligosacchariden vorhanden sein, wobei die erste derartige Einheit zur Gundeinheit der allgemeinen Formel I gehört und die bis zu 19 weiteren derartigen Einheiten der allgemeinen Formel II entsprechen. Die erfindungsgemäßen Oligosaccharide können somit geradkettige oder verzweigte Moleküle darstellen.

Nimmt man einmal für Erläuterungszwecke an, dass bei obigen Formeln (III) bis (VII) keiner der Reste R ein Verknüpfung zur nächsten [Gal - HexNAc] - Einheit bedeutet, dann besitzen die Verbindungen der Formel (III) und (IV) jeweils nur eine endständige [Gal - HexNAc] - Einheit, nämlich die am weitestens links gezeigte, während die anderen Einheiten mittelständig sind. Die Verbindungen der Formeln (V), (VI) und (VII) besitzen jeweils zwei derartige endständige Einheiten, wobei die Verbindung (V) auch zwei und die Verbindung (VII) eine mittelständige Einheit der allgemeinen Formel II aufweisen. Bei diesen endständigen Einheiten können die Reste R auch einen Deoxyhexose-Rest darstellen. Somit können eine, zwei, drei (sofern vorhanden) oder alle endständigen [Gal - HexNAc] - Einheiten der erfindungsgemäßen Oligosaccharide einen derartigen Deoxyhexose-Rest oder zwei derartige Reste aufweisen.

Zudem können eine oder mehrere (sofern vorhanden) endständige [Gal-HexNAc]-Einheit(en), bei der oder bei denen der oder die Rest(e) R einen Deoxyhexose-Rest bedeutet(n), an der HexNAc-Monosaccharid-Einheit einen weiteren Deoxyhexose-Rest aufweisen.

Zu den erfindungsgemäßen Oligosacchariden zählen vorzugsweise solche, die eine, zwei, drei oder auch alle vier der folgenden Kriterien erfüllen:
- der Deoxyhexose-Rest, sofern vorhanden, der Galactose-Monosaccharid-Einheit der endständigen [Gal-HexNAc]-Einheit(en) ist α1-2 glycosidisch damit verknüft,
- bei dem Deoxyhexose-Rest, sofern vorhanden, der Galactose-Monosaccharid-Einheit der endständigen [Gal-HexNAc]-Einheit(en) handelt es sich um einen Rhamnose- und Fucose-Rest
- der weitere Deoxyhexose-Rest, sofern vorhanden, der HexNAc-Monosaccharid-Einheit der endständigen [Gal-HexNAc]-Einheit(en) ist α1-3 oder α1-4 glykosidisch damit verknüpft
- bei dem weiteren Deoxyhexose-Rest, sofern vorhanden, der HexNAc-Monosaccharid-Einheit der endständigen [Gal-HexNAc]-Einheit(en) handelt es sich um einen Rhamnose- und Fucose-Rest.

Bei den erfindungsgemäßen Oligosacchariden handelt es sich im übrigen um bekannte Verbindungen. So zählen die erfindungsgemäß eingesetzten Oligosaccharide LNT und LNFP I zu den Oligosacchariden, die in Humanmilch vorhanden sind.

Es wurde nun überraschend gefunden, dass die erfindungsgemäßen Oligosaccharide die Invasion und Infektion von Säugerzellen durch Listerien zumindest verringern oder sogar verhindern und zur Bekämpfung von durch derartige Listerien verursachte Erkrankungen eingesetzt werden können.

Es wurde beispielsweise gefunden, dass die erfindungsgemäßen Oligosaccharide die Invasion und Infektion von Säugerzellen durch Listerien, insbesondere Listeria monocytogenes verhindern können. Die Ergebnisse der durchgeführten Untersuchungen zeigen deutlich, dass weder der Vorgang der Phagozytose an sich, noch die Replikation der aufgenommenen Listerien gehemmt wird. Als besonders stark inhibitorisch erwies sich unter anderem das erfindungsgemäße Oligosaccharid LNFP I.

Das Pentasaccharid LNFP I zeigte somit eine starke, allerdings dosisabhängige Hemmwirkung gegen Listerien. Strukturisomere Verbindungen zu LNFP I, wozu beispielsweise die Oligosaccharide LNFP II und LNFP III sowie LNFP V gehören, zeigten dagegen keine derartige Wirkung. Die Oligosaccharide LNFP II und LNFP III finden sich im übrigen ebenso wie das LNFP I in der Humanmilch.

Die erfindungsgemäßen Oligosaccharide können nicht nur als freie Oligosaccharide (d.h. mit einem reduzierenden Ende), sondern auch an einen Träger immobilisiert oder adsorbiert eingesetzt werden. Bei diesem Träger kann es sich um ein Peptid/Protein (beispielsweise BSA), ein Lipid, (Glycolipid, Ceramid), ein Polymer oder ein Biopolymer (beispielsweise Kohlenhydrat-Dendrimer, Polysaccharid, Polyacrylamid) oder jedes andere Aglykon handeln.

Die erfindungsgemäßen Oligosaccharide, seien es nun freie Oligosaccharide oder an einen Träger gebundene Oligosaccharide, können verschiedenen Nahrungsmitteln, diätetischen Mitteln und pharmazeutischen Mitteln einverleibt sein. Alle diese Mittel können in flüssiger oder fester Form vorliegen. Der hier verwendete Begriff Nahrungsmittel umfasst nicht nur die eigentliche Nahrungsmittel sondern auch Nahrungsergänzungsmittel, Getränke sowie Nahrungen einschließlich Säuglings- und Babynahrungen. Der Begriff Babynahrung bzw. Säuglingsnahrung umfasst alle künstlich hergestellten Nahrungen, jedoch keine Humanmilch. Als "künstlich" werden hier solche Nahrungen verstanden, die aus Rohstoffen pflanzlichen und tierischen Ursprungs, jedoch nicht humanen Ursprungs, hergestellt werden. Diese Nahrungsmittel können auf beliebige Weise an einen Menschen oder ein Tier verabreicht werden. Dazu zählt auch die Verabreichung als Sondennahrung in den Magen.

Die erfindungsgemäßen Oligosaccharide können beispielsweise als Beimengungen oder Additive folgenden Produkten zugegeben werden, wobei diese Aufzählung nicht abschließend ist: Milch und Milchprodukte, Säuglings- und Kindernahrungen, Schokoriegel, Joghurtgetränke, Käse-, Wurst- und Fleischwaren, Aufbaunahrung, Sondennahrung und Produkten für Schwangere.

Die erfindungsgemäßen Oligosaccharide können auch in Form eines pharmazeutischen Mittels alleine oder zusammen mit einem oder mehreren zusätzlichen Wirkstoff(en) verabreicht werden. Sie können beispielsweise als Tablette/Sachet formuliert sein. Für die Formulierung derartiger Pharmazeutika können übliche Adjuvantien, Träger, Hilfsmittel Verdünnungsmittel, feucht-haltende Mitteln, Verdickungsmittel, Geschmacksstoffe, Süßungsmittel usw. Anwendung finden.

Die pharmazeutischen Mittel können auf jede übliche Weise an einen Patienten ( d.h. Mensch und Tier) verabreicht werden. Zweckmäßigerweise handelt es sich jedoch um Mittel, die zur oralen, lingualen, nasalen, intestinalen, bronchialen, vaginalen, topischen (Haut und Schleimhaut) und per os Verabreichung geeignet und entsprechend der Verabreichungsart formuliert sind.

Die mindestens ein erfindungsgemäßes Oligosaccharide enthaltenden Nahrungsmittel, diätetischen Mittel und pharmazeutischen Mittel können unter anderem zur Prävention und Behandlung von Infektionen des Gastrointestinaltraktes, beispielsweise bei Listeriosen, des Blutsystemes, der Atemwege, des Urogenitaltraktes sowie des Nasen-Rachenraumes und zum Schutz von Endothelien, Epithelien und Mukosa eingesetzt werden. Sie können somit auch topisch auf die Haut aufgetragen oder auch auf Schleimhäuten Anwendung finden. Zu diesen Schleimhäuten zählen nasale, intestinale, bronchiale und vaginale Schleimhäute. So können die erfindungsgemäßen Oligosaccharide beispielsweise einem Mundspülmittel beigefügt sein. Als Zielgruppen für die erfindungsgemäßen Oligosaccharide können alle Altersstufen von Neugeborenen bis zu Senioren genannt werden. Besondere Einsatzgebiete sind der Schutz und die Behandlung von Schwangeren, Kranken, geschwächten und älteren Menschen, bei denen die Vermeidung beispielsweise einer Listeriose von besonderer Bedeutung ist.

Die erfindungsgemäßen Oligosaccharide und zum Beispiel LNFP I werden nach geeigneten, bekannten Verfahren chemisch, enzymatisch oder in einer Kopplung beider Technologien hergestellt. Diese erfolgt systematisch aus den Monosaccharidbestandteilen oder durch Modifzierung geeigneter Oligosaccharid-Rohstoffe. Bei den enzymatischen Synthesen werden sowohl Transferasen (Leloir, bzw. nicht-Leloir) als auch Hydrolasen (reverse Hydrolyse bzw. Transglycosylierung) eingesetzt. Die Enzyme können dabei sowohl frei als auch eingebunden (etwa Membranreaktor) oder kovalent an einen Träger (beispielsweise beads, Chromatograpahiematerial oder Filtrationsmembranen) gebunden sein. Es ist auch möglich, pro- oder eukaryontische Zellen zur Synthese einzusetzen, sofern diese Zellen die geeigneten Enzyme aufweisen. Bezüglich weiterer Einzelheiten der Herstellung der erfindungsgemäßen Oligosaccharide wird beispielsweise verwiesen auf Carbohydrates in

Chemistry and Biology (Herausgeber Ernst, Hart, Sinay) Wiley VCH-Weinheim 2000 Vol. I-IV).

Beispielhafte Diätetika und Pharmazeutika, welche die erfindungsgemäßen Oligosaccharide enthalten, sind nachstehend aufgeführt.

### Beispiel 1:

Zur Herstellung von Sachets werden jeweils 100 mg LNFP I mit 990 mg Maltodextrin trocken gemischt und dann in Sachets verpackt. Diese Sachets werden dreimal täglich zu den Mahlzeiten verabreicht.

### Beispiel 2:

Eine bekannte Heilnahrung (d.h. Milupa® HN 25, bilanzierte Diät) in Form eines Perlates mit 18,8 g Eiweiß, 8,6 g Fett, 62,8 g Kohlenhydrate, 3,3 g Mineralstoffen und Vitaminen wird bei der per se bekannten Herstellung mit LNFP I in einer solchen Menge versetzt, dass 50 mg LNFP I in 100 g des fertigen Perlats enthalten sind.

Für die Herstellung einer flüssigen Heilnahrung werden 100 ml der bekannten Heilnahrung *Milupa HN 25 flüssig* (2,3 g Eiweiß, 1,6 g Fett, 8,5 g Kohlenhydrate, 37 g Mineralstoffe, Vitamine) mit 7 mg LNFP I versetzt.

### Beispiel 3:

### Produkt für Schwangere

Eine Brausetablette (Endgewicht 4,15 g) (*Neovin*® von Milupa) wird unter Beimengung von 200 bis 500 mg LNFP I auf per se bekannte Weise hergestellt. Täglich wird eine Tablette in 150 ml Wasser gelöst und getrunken.

### Beispiel 4:

### Produkt für Ältere und geschwächte Personen

Eine bilanzierte pulverförmige Aufbaunahrung (*Dilsana*® von Milupa) mit 22,5 g Eiweiß, 7,7 g Fett, 60,8 g Kohlenhydrate, 5,4 g Mineralstoffen und Vitaminen wird auf per se bekannte Weise unter Einarbeitung von 100 mg bis 1000 mg LNFP I pro 100 g Pulver hergestellt. Täglich werden bis zu 3 x 50 g der Nahrung in 150 ml Wasser gelöst und verabreicht.

### Beispiel 5

### Tee

100 g eines auf übliche Weise hergestellten Instant-Tee-Pulvers werden mit 2 g LNFP I vermengt. Je 3,8 g Teepulver werden in 100 ml heißem Wasser gelöst und dreimal täglich verabreicht.

### Beispiel 6

Eine im Eiweiß adaptierte Säuglingsmilchnahrung (*Aptamil*® von Milupa) mit 11,8 g Eiweiß, 56,9 g Kohlenhydrate24,9 g Fett, 2,5 g Mineralstoffen, Vitaminen und 45 mg Taurin wird auf übliche Weise in Form eines Perlates hergestellt, das mit 100 mg bis 1000 mg LNFP I pro 100 g Säuglingsmilchnahrung vermengt wird.

## Patentansprüche

1. Verwendung von neutralen geradkettigen oder verzweigten Oligosacchariden, die aus einer Grundeinheit der folgenden allgemeinen Formel I: 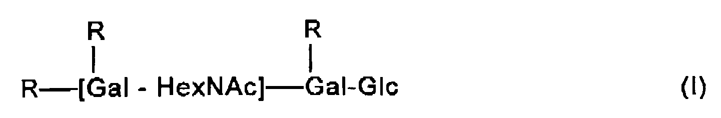 und 0 bis 19 weiteren damit direkt oder indirekt verknüpften Einheiten der folgenden allgemeinen Formel II 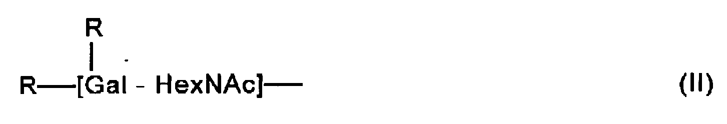 worin
Gal eine Galactose-Monosaccharid-Einheit,
Glc eine Glucose-Monosaccharid-Einheit,
HexNAc eine N-acetylierte Galactosamin- oder Glucosamin-Monosaccharid-Einheit (GalNAc bzw. GlcNAc) bedeuten,
die Reste R jeweils unabhängig voneinander eine β 1-3 oder β 1-6 glykosidische Verknüpfung zur HexNAc-Monosaccharid-Einheit der nächsten [Gal-HexNAc]-Einheit der allgemeinen Formel II und an einer endständigen [Gal-HexNAc]-Einheit einen mit der Galactose-Monosaccharid-Einheit dieser endständigen [Gal-HexNAc]-Einheit verknüpften Deoxyhexose-Rest darstellen oder nicht vorhanden sind, und
eine endständige [Gal-HexNAc]-Einheit, bei der der oder die Rest(e) R einen Deoxyhexose-Rest bedeutet(n), an der HexNAc-Monosaccharid-Einheit einen weiteren Deoxyhexose-Rest aufweisen kann, zusammengesetzt sind,
zur Herstellung eines Nahrungsmittels, diätetischen Mittels und pharmazeutischen Mittels zur Verringerung oder Verhinderung der Invasion und Infektion von Säugerzellen durch Listerien und zur Bekämpfung von durch Listerien verursachten Erkrankungen.

2. Verwendung nach Anspruch 1,
**dadurch gekennzeichnet, dass**
die Oligosaccharide der folgenden allgemeinen Formel entsprechen 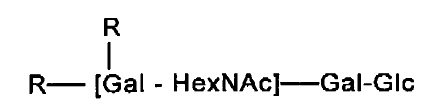 worin die Reste R die oben angegebenen Bedeutungen besitzen.

3. Verwendung nach Anspruch 1 oder 2,
**dadurch gekennzeichnet, dass**
die Oligosaccharide mindestens eine der folgenden Kriterien erfüllen:
- der Deoxyhexose-Rest, sofern vorhanden, der Galactose-Monosaccharid-Einheit der endständigen [Gal-HexNAc]-Einheit ist α1-2 glycosidisch damit verknüft,
- bei dem Deoxyhexose-Rest, sofern vorhanden, der Galactose-Monosaccharid-Einheit der endständigen [Gal-HexNAc]-Einheit handelt es sich um einen Rhamnose- und Fucose-Rest
- der weitere Deoxyhexose-Rest, sofern vorhanden, der HexNAc-Monosaccharid-Einheit der endständigen [Gal-HexNAc]-Einheit ist α1-3 oder α1-4 glykosidisch damit verknüpft
- bei dem weiteren Deoxyhexose-Rest, sofern vorhanden, der HexNAc-Monosaccharid-Einheit der endständigen [Gal-HexNAc]-Einheit handelt es sich um einen Rhamnose- und Fucose-Rest.

4. Verwendung nach Anspruch 1,
**dadurch gekennzeichnet, dass**
als Oligosaccharid Lacto-N-tetraose
(Gal βl-3GlcNAcβ 1-3Galβ 1-4Glc, LNT) eingesetzt wird.

5. Verwendung nach Anspruch 4,
**dadurch gekennzeichnet, dass**
die Lacto-N-tetraose in α1-2 glycosidischer Verknüpfung an der endständigen Galactose - Monosaccharid - Einheit durch Fucose derivatisiert ist und es sich um Lacto-N-fucopentaose I (LNFP I) handelt.

6. Verwendung nach einem der vorhergehenden Ansprüche,
**dadurch gekennzeichnet, dass**
die Oligosaccharide an einem inerten Träger immobilisiert oder adsorbiert sind.

7. Verwendung nach Anspruch 6,
**dadurch gekennzeichnet, dass**
es sich bei dem Träger um ein Peptid, ein Protein, ein Lipid, ein Polymer oder ein Biopolymer handelt.

8. Verwendung nach einem der vorhergehenden Ansprüche,
**dadurch gekennzeichnet, dass**
die Oligosaccharide einem flüssigen oder festen Nahrungsmittel (mit Ausnahme von Humanmilch), diätetischen Mittel oder pharmazeutischen Mittel zur Verabreichung an einen Menschen oder ein Tier einverleibt sind oder zur Herstellung eines derartigen Mittels dienen.

9. Verwendung nach Anspruch 8,
**dadurch gekennzeichnet, dass**
es sich bei dem Nahrungsmittel um eine Säuglings- oder Kindernahrung handelt.

10. Verwendung nach Anspruch 8,
**dadurch gekennzeichnet, dass**
das pharmazeutische Mittel zur oralen, lingualen, nasalen, bronchialen, vaginalen, topischen (Haut und Schleimhaut) oder per os oder zur Verabreichung mittels einer Sonde in den Magen eines Menschen oder eines Tieres dient.

11. Verwendung nach einem der vorhergehenden Ansprüche,
**dadurch gekennzeichnet, dass**
die Oligosaccharide in einer Menge von mindestens 1 mg pro kg Körpergewicht und Tag einem Menschen oder einem Tier verabreicht werden, jedoch nicht in Form von Humanmilch.

12. Verwendung eines pharmazeutischen Mittels nach einem der vorhergehenden Ansprüche,
**dadurch gekennzeichnet, dass**
das pharmazeutische Mittel zusätzlich einen üblichen Hilfsstoff oder mehrere übliche Hilfsstoffe, wozu Verdünnungsmittel, feucht-haltende Mitteln, Verdickungsmittel, Geschmacksstoffe, Süßungsmittel und Träger zählen, sowie auch einen weiteren Wirkstoff oder mehrere weitere Wirkstoffe enthalten kann.

13. Verwendung nach einem der vorhergehenden Ansprüche,
**dadurch gekennzeichnet, dass**
das Nahrungsmittel, diätetische Mittel oder pharmazeutische Mittel in einer solchen Menge an einen Menschen oder ein Tier, jedoch nicht in Form von Humanmilch, verabreicht wird, dass mindestens 1 mg Oligosaccharid nach einem der Ansprüche 1 bis 7 pro kg Körpergewicht und Tag dem Mensch oder Tier zugeführt wird.

## Claims

1. Use of neutral straight-chain or branched oligosaccharides of a base unit of the following general formula I: 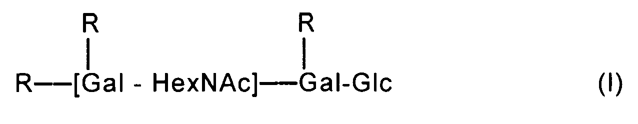 and 0 to 19 further units directly or indirectly linked thereto and having the following general formula II: 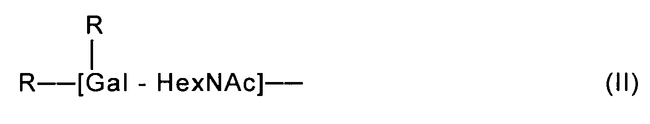 wherein
Gal represents a galactose monosaccharide unit,
Glc represents a glucose monosaccharide unit,
HexNAc represents an N-acetylated galactosamine monosaccharide or glucosamine monosaccharide unit (GalNAc or GlcNAc),
the residues R represent, or are not present, as the case may be, in each case independently of each other, a β 1-3 or β 1-6 glycosidic bond to the HexNAc monosaccharide unit of the next [Gal-HexNAc] unit of the general formula II, and, at a terminal [Gal-HexNAc] unit, a deoxyhexose residue linked to the galactose monosaccharide unit of said terminal [Gal-HexNAc] unit, and
a terminal [Gal-HexNAc] unit, wherein the residue(s) R represent(s) a deoxyhexose residue, at which the HexNAc monosaccharide unit may have a further deoxyhexose residue,
for producing a food, dietetic composition and apharmaceutical composition for reducing or preventing the invasion and infection of mammal cells by listeria bacteria and for combating diseases caused by listeria bacteria.

2. Use according to claim 1,
**characterized in that**
the oligosaccharides correspond to the following general formula: 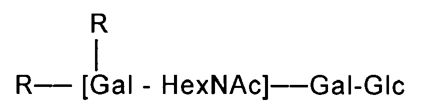 wherein the residues R have the meanings indicated above.

3. Use according to claim 1 or 2,
**characterized in that**
the oligosaccharides meet at least one of the following criteria:
- the deoxyhexose residue, if present, of the galactose monosaccharide unit of the terminal [Gal - HexNAc] unit is linked to it in an α1-2 glycosidic bond,
- the deoxyhexose residue, if present, of the galactose monosaccharide unit of the terminal [Gal - HexNAc] unit is a rhamnose or fucose residue,
- the further deoxyhexose residue, if present, of the HexNAc monosaccharide unit of the terminal [Gal - HexNAc] unit is linked to it in an α 1-3 or α 1-4 glycosidic bond,
- the further deoxyhexose residue, if present, of the HexNAc monosaccharide unit of the terminal [Gal - HexNAc] unit is a rhamnose or fucose residue.

4. Use according to claim 1,
**characterized in that**
lacto-N-tetraose (Gal β1-3GlcNAcβ 1-3Gal β1-4Glc, LNT) is used as the oligosaccharide.

5. Use according to claim 4,
**characterized in that**
the lacto-N-tetraose is derivatized in an α 1-2 glycosidic bond at the terminal galactose monosaccharide unit by means of fucose, and is lacto-N-fucopentaose I (LNFP I).

6. Use according to any one of the preceding claims,
**characterized in that**
the oligosaccharides are immobilized on or adsorbed in an inert carrier.

7. Use according to claim 6,
**characterized in that**
the carrier is a peptide, a protein, a lipid, a polymer or a bio-polymer.

8. Use according to any one of the preceding claims,
**characterized in that**
the oligosaccharides are incorporated in a liquid or solid food (with the exception of human milk), dietetic composition or pharmaceutical composition for administration to a human or an animal, or serve in the production of such an agent.

9. Use according to claim 8,
**characterized in that**
the food is a baby food or an infant formula.

10. Use according to claim 8,
**characterized in that**
the pharmaceutical composition is suitable for oral, lingual, nasal, bronchial, vaginal, topical (skin and mucosa) or per os administration, or for administration by means of a probe into the stomach of a human or an animal.

11. Use according to any one of the preceding claims,
**characterized in that**
the oligosaccharides are administered in an amount of at least 1 mg per kg of body weight and per day to a human or an animal, but not in the form of human milk.

12. Use of a pharmaceutical composition according to any one of the preceding claims,
**characterized in that**
the pharmaceutical composition may contain in addition an usual auxiliary agent or several usual auxiliary agents including diluents, moisturizing agents, thickening agents, flavouring agents sweetening agents and carries, as well as one additional active agent or several further active agents.

13. Use according to any one of the preceding claims,
**characterized in that**
the food, the dietetic composition or the pharmaceutical composition is administered in such an amount to a human or an animal, but not in the form of human milk, that at least 1 mg oligosaccharide according to one of the claims 1 to 7 is delivered to the human or animal per kg of body weight and per day.

## Revendications

1. Utilisation d'oligosaccharides linéaires ou ramifiés neutres, qui sont composés d'un motif structurel de formule générale I suivante : 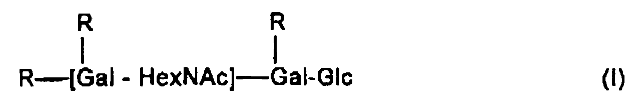 et de 0 à 19 autres motifs, directement ou indirectement liés à celui-ci, de formule générale II suivante 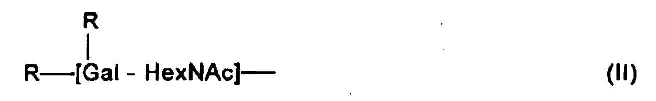 dans lesquelles
Gal est un motif du monosaccharide galactose,
Glc est un motif du monosaccharide glucose,
HexNAc est un motif du monosaccharide galactosamine ou glucosamine N-acétylée (GalNAc ou GlcNAc),
les groupes R représentent chacun, indépendamment les uns des autres, une liaison β-1-3- ou β-1-6-glycosidique avec le motif du monosaccharide HexNAc du motif [Gal-HexNAc] suivant de formule générale II et, au niveau d'un motif [Gal-HexNAc] terminal, un groupe désoxyhexose lié au motif du monosaccharide galactose de ce motif [Gal-HexNAc] terminal, ou n'existent pas, et
un motif [Gal-HexNAc] terminal, dans lequel le ou les groupes R représentent un ou des groupes désoxyhexose, peut présenter, au niveau du motif du monosaccharide HexNAc, un autre groupe désoxyhexose,
pour la préparation d'un produit alimentaire, d'un produit diététique et d'un produit pharmaceutique destiné à diminuer ou à empêcher l'invasion et l'infection de cellules de mammifères par des Listeria et pour lutter contre des maladies causées par des Listeria.

2. Utilisation selon la revendication 1,
**caractérisée en ce que**
les oligosaccharides correspondent à la formule générale suivante 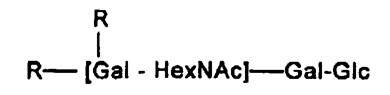 dans laquelle les groupes R ont les significations susmentionnées.

3. Utilisation selon la revendication 1 ou 2,
**caractérisée en ce que**
les oligosaccharides remplissent au moins l'un des critères suivants :
- le groupe désoxyhexose, s'il est présent, du motif du monosaccharide galactose du motif [Gal-HexNAc] terminal est relié à celui-ci par une liaison α-1-2-glycosidique,
- le groupe désoxyhexose, s'il est présent, du motif du monosaccharide galactose du motif [Gal-HexNAc] terminal est un groupe rhamnose et fucose,
- l'autre groupe désoxyhexose, s'il est présent, du motif du monosaccharide HexNac du motif [Gal-HexNAc] terminal est relié à celui-ci par une liaison α-1-3- ou α-1-4- glycosidique,
- l'autre groupe désoxyhexose, s'il est présent, du motif du monosaccharide HexNAc du motif [Gal-HexNAc] terminal est un groupe rhamnose et fucose.

4. Utilisation selon la revendication 1,
**caractérisée en ce que**
l'on met en oeuvre, en tant qu'oligosaccharide, le lacto-N-tétraose
(Gal β1-3GlcNAcβ 1-3Galβ 1-4Glc, LNT).

5. Utilisation selon la revendication 4,
**caractérisée en ce que**
le lacto-N-tétraose, lié par une liaison α-1-2-glycosidique, est dérivé, au niveau du motif du monosaccharide galactose terminal, par le fucose et il s'agit de lacto-N-fucopentaose I (LNFP I).

6. Utilisation selon l'une quelconque des revendications précédentes,
**caractérisée en ce que**
les oligosaccharides sont immobilisés ou adsorbés sur un support inerte.

7. Utilisation selon la revendication 6,
**caractérisée en ce que**
le support est un peptide, une protéine, un lipide, un polymère ou un biopolymère.

8. Utilisation selon l'une quelconque des revendications précédentes,
**caractérisée en ce que**
les oligosaccharides sont incorporés dans un produit alimentaire liquide ou solide (à l'exception du lait humain), un produit diététique ou un produit pharmaceutique destiné à être administré à un être humain ou à un animal, ou servent à fabriquer un tel produit.

9. Utilisation selon la revendication 8,
**caractérisée en ce que**
le produit alimentaire est un aliment pour nourrisson ou pour enfant.

10. Utilisation selon la revendication 8,
**caractérisée en ce que**
le produit pharmaceutique est destiné à une administration par voie orale, linguale, nasale, bronchique, vaginale, topique (peau et muqueuses) ou per os, ou à une administration par l'intermédiaire d'une sonde dans l'estomac d'un être humain ou d'un animal.

11. Utilisation selon l'une quelconque des revendications précédentes,
**caractérisée en ce que**
les oligosaccharides sont administrés à un être humain ou à un animal à raison d'au moins 1 mg par kg de poids corporel et par jour, mais pas sous la forme de lait humain.

12. Utilisation d'un produit pharmaceutique selon l'une quelconque des revendications précédentes,
**caractérisée en ce que**
le produit pharmaceutique peut contenir en plus un excipient classique ou plusieurs excipients classiques, parmi lesquels des diluants, des agents humidifiants, des épaississants, des aromatisants, des édulcorants et des véhicules, ainsi qu'un autre principe actif ou plusieurs autres principes actifs.

13. Utilisation selon l'une quelconque des revendications précédentes,
**caractérisée en ce que**
le produit alimentaire, le produit diététique ou le produit pharmaceutique est administré à un être humain ou à un animal, mais pas sous la forme de lait humain, dans une quantité telle qu'au moins 1 mg d'oligosaccharide selon l'une quelconque des revendications 1 à 7 par kg de poids corporel et par jour est administré à l'être humain ou à l'animal.
